Europäisches Patentamt

**European Patent Office**

Office européen des brevets

(11) Publication number:

**0 264 280**
**A2**

(12) **EUROPEAN PATENT APPLICATION**

(21) Application number: **87309114.4**

(22) Date of filing: **15.10.87**

(51) Int. Cl.⁴: **C 07 C 7/152**
**C 07 C 7/11**

(30) Priority: **16.10.86 US 919745**

(43) Date of publication of application:
**20.04.88 Bulletin 88/16**

(84) Designated Contracting States:
**AT BE DE FR GB IT NL**

(71) Applicant: **BETZ EUROPE, INC.**
**4636 Somerton Road**
**Trevose Pennsylvania 19047 (US)**

(72) Inventor: **Roling, Paul Vincent**
**4323 Chestergate**
**Spring, TX 77373 (US)**

(74) Representative: **Gore, Peter Manson et al**
**W.P. THOMPSON & CO. Coopers Building Church Street**
**Liverpool L1 3AB (GB)**

(54) **Inhibition of fouling during washing under basic conditions.**

(57) A method of inhibiting the formation and deposition of fouling materials in a basic solution in the washing of hydrocarbon streams. The method entails adding to the basic wash a sufficient amount of water-soluble sulfite or bisulfite to inhibit fouling due to polymer formation of oxygenated hydrocarbon components.

EP 0 264 280 A2

**Description**

INHIBITION OF FOULING DURING WASHING UNDER BASIC CONDITIONS

The present invention relates to the prevention of fouling under those conditions which are basic in nature, particularly in a basic solution which is in contact with a gaseous or liquid hydrocarbon stream.

In cracking operations (pyrolysis) such as, for example, in the cracking (pyrolysis) of ethane, propane, and naphthas to olefins, oxygenated compounds, including carbonyl compounds, are formed. The amount of carbonyl compounds, such as aldehydes and ketones, formed in such an operation can vary widely, but it typically about 1 to 100 ppm in the gas stream with concentrations as high as 1000 ppm occasionally being encountered because of the utilization of the various feedstocks and cracking temperatures. When the gas stream is passed through a wash which is basic, namely pH greater than 7, to remove acidic components such as, for example, hydrogen sulfide and carbon dioxide, oxygenated compounds and carbonyl compounds are also removed. These oxygen compounds, particularly acetaldehyde, will, however, undergo polymerization in the presence of the base. In the wash tower, the polymer will settle on the trays leading to fouling and eventual plugging of the trays, which means the unit must be shut down to clean the trays, which is obviously a costly operation. The type of basic wash systems where treatment is required to inhibit fouling include amine acid gas scrubber (e.g., MEA, DEA, isopropylamine or butyl amine) and caustic wash systems.

US- A- 3 336 414 and US- A- 3 308 201 disclose processes utilizing aqueous caustic washes (pH > 10) of carbonyl-contaminated hydrocarbons of remove carbonyl compounds.

In US- A- 3 218 489, carbonyl compounds, aldehydes are removed from a butadiene stream (obtained from the pyrolysis of saturated hydrocarbons) by selective hydrogenation to reduce some of the carbonyls. This procedure is followed by caustic washing to remove substantially all of the remaining carbonyl compounds. If caustic washing is performed before the hydrogenation, carbonyl polymeric materials from Aldol condensation foul the process equipment.

US- A- 3 801 669 discloses the use of Portland cement to extract carbonyl compounds from hydrocarbon streams.

At acid pH, extraction of carbonyl compounds from hydrocarbon streams by aqueous solutions of hydrazine compounds is reported in US- A- 3 793 187. The carbonyl compounds need to be extracted because they have an inhibiting effect on further processing steps. Only extractions of liquid systems are contemplated in US- A- 3 793 187.

In US- A- 3 535 399, carbonyl compounds are removed from gaseous hydrocarbon streams by contacting the streams with an aqueous solution of sodium hydroxide and urea. The caustic removes acid materials and the urea complexes with the carbonyl compounds to form aldehyde-urea of ketone-urea resins that are reportedly entrained in the aqueous solution.

Substituted hydroxylamine compounds such as, for example, N, N-diethylhydroxylamine are used to prevent polymerization of unsaturated aldehydes in dilute alcohol solutions, but not of the neat unsaturated aldehydes in US- A- 3 849 498. Copending Application 86 307642.8 is directed to certain hydroxylamines as inhibitors for carbonyl induced fouling in the enviroment of the present invention

DE- B- 1 072 607 discloses that polystyrene based cation exchange resins can be treated with a solution of hydroxylamine hydrochloride to remove carbonyl compounds from sulfite liquor.

FR- B- 1 546 472 discloses a procedure of treating a carbonyl-contaminated glycerol with an acid and 2,4-dinitro-phenyl hydrazine.

Sulfites have been used to scavenge oxygen in aqueous systems and, in particular, boiler water systems to avoid oxygen corrosion of the metallic structures.

After reviewing the problems associated with carbonyl contamination of hydrocarbons, particularly the gaseous olefins derived from pyrolytic cracking, it was apparent that there was a problem in the cracking industry in as much as it required a treatment which would control the formation and deposition of fouling materials during the basic wash of hydrocarbons. Most desirably, the treatment would be such that it would operate effectively in the highly basic wash to alleviate the potential problems due to the oxygenated compounds, particularly the carbonyls, without the formation of other solid materials which had to be removed. The treatment not only had to be effective but also cost-effective.

It has now been found possible to inhibit the formation and deposition of fouling materials during the basic wash and in particular the caustic wash of hydrocarbons containing oxygenated compounds, and in particular the gaseous olefins containing carbonyl compounds. Such carbonyl compounds under alkaline conditions undergo in many instances Aldol condensation reactions to produce polymeric materials which deposit on the equipment and in particular plug the trays in the caustic wash tower.

According to the present invention there is provided a method for inhibiting the formation and deposition of fouling materials during the basic washing of hydrocarbons contaminated with at least one oxygenated compound which comprises performing the wash of the hydrocarbon in the presence of sufficient amount for the purpose of a water-soluble sulfite or bisulfite compound.

The method of the present invention is particularly applicable to inhibiting the formation of such fouling materials and their deposition of the structural parts of the equipment used in the wash under basic conditions.

The method of the present invention is particularly appropriate for the basic washing process which follows the pyrolytic cracking of such hydrocarbons such as, for example, ethane, propane, butane, naphtha and mixtures thereof to produce the corresponding gaseous olefin, such as, for example, ethylene, propylene or butadiene, containing the carbonyl as well as other contaminants.

Generally the basic washing entails contacting in wash towers an aqueous basic solution with the gaseous olefins to remove any hydrogen sulfide, carbon dioxide and other oxygenated compounds. As earlier discussed the conditions are such as to be conducive for condensation reactions of any aldehydes/ketones (acetaldehydes) contained therein.

Usually the hydrocarbon being washed is in the gaseous state.

The method of the present invention generally entails ensuring that the basic wash takes place in the presence of a water-soluble sulfite or bisulfite compound (or mixtures thereof).

Examples of the sulfite of bisulfite include alkali metal compounds eg., sodium sulfite, sodium bisulfite, potassium sulfite, potassium bisulfite, ammonium sulfite, and ammonium bisulfite.

These fouling inhibitors can be added to the caustic tower as neat materials or as solutions and should be added in an amount sufficient to inhibit carbonyl induced fouling. The preferred method of addition is as an aqueous solution with 2 to 50 weight percent inhibitor present, so that accurate metering of the inhibitor to the tower can be achieved. The fouling inhibitors can be used in a continuous or batch process.

It is theorized that the inhibitors prevent fouling by forming a complex with the carbonyl compounds and that the complex does not undergo polymerization. For one mole of carbonyl compound, one mole of inhibitor is needed. However, since other unknown side reactions could consume the inhibitor, a molar ratio greater than 1:1 should be used. In general, a molar ratio of 1:1 to 10:1 of inhibitor to carbonyl content should suffice, with a preferred ratio of 1:1 to 3:1. A ratio of substantially 1:1 may be employed.

A preferred formulation of the sulfite compound for addition to the basic wash would on a weight basis comprise

12% sulfite or bisulfite compound
88% water

This product would be added to the wash in quantities to assure that the molar ratio of sulfite to oxygenated or carbonyl compound is 1:1 or greater. Treatment ranges of from about 1 to 10,000 parts of product per million of wash solution could be utilized.

The following Examples are given to illustrate the invention, but are not meant to be limiting. The test conditions in the examples are accelerated conditions to show the effects of the inhibitors in a short period of time instead of the three months or more of the field conditions.

Example A

Into a round-bottomed flask equipped with a magnetic stirrer and a reflux condenser, there were introduced 95 mL of water and 5g (0.11 mol) of acetaldehyde. This mixture was refluxed for one hour. At the end of this period no polymer was observed.

Example B

Example A was repeated but the water was made to a pH of 3.5 by addition of hydrochloric acid. Again at the end of one hour no polymer was noted.

Example C

As in Example A, there was refluxed 5g (0.125 mol) of sodium hydroxide, 90 mL of water, and 5g of acetaldehyde for one hour. An orange solution and polymer resulted. The polymer was filtered, air-dired, and weighed. The polymer weighed 3.7g. In three other runs there resulted 4g, 5g, and 2g of polymer.

In the above three comparative examples, it is shown that under neutral (Example A) or acid (Example B) conditions the polymer did not form. However, under the caustic conditions of Example C, a large amount of polymer resulted.

Example D

The procedure of Example A was followed with the use of 5g of sodium hydroxide, 7g (0.12 mol) of urea, 83 mL of water and 5g of acetaldehyde. These ingredients were refluxed for one hour. There resulted 1.5g of polymer.

Example E

As in Example A, 5g of sodium hydroxide, 10g (0.12 mol) of N,N-diethylhydroxylamine, 80 mL of water and 5g of acetaldehyde were refluxed for one hour. The resulting polymer weighed 4g.

Example F

As in Example A, 5g of sodium hydroxide, 7g (0.12 mol) of monoethanolamine, 83 mL of water and 5g of acetaldehyde were refluxed for one hour. The polymer that resulted weighted 1g.

Examples D,E, and F illustrate that the prior art methods of urea (Example D) and of substituted hydroxylamines (Example E) and monoethanolamine do not prevent polymer formation under the basic conditions.

Example G

As in Example A, 5g of sodium hydroxide, 13g (0.12 mol) of hydroquinone, 77 mL of water and 5g of acetaldehyde were refluxed for one hour. There resulted 7.5g of polymer. This example established that the polymer formation is not inhibited by traditional antioxidant scavenger-reducing compounds.

Example H

As in Example A, 5g of sodium hydroxide, 15g (0.12 mol) of sodium sulfite, 75 mL of water and 5g of acetaldehyde were refluxed for one hour. The solution turned red, but no polymer was formed. A second reaction gave the same result.

Example H illustrated the efficacy of the present invention. Potassium and ammonium sulfite, as well as sodium, potassium and ammonium bisulfite, are similarly effective.

## Claims

1. A method for inhibiting the formation and deposition of fouling materials during the basic washing of hydrocarbons contaminated with at least one oxygenated compound which comprises performing the wash of the hydrocarbon in the present of sufficient amount for the purpose of a water-soluble sulfite or bisulfite compound.

2. A method according to claim 1 for inhibiting the formation and deposition of fouling materials on the structural parts of the basic washing equipment, wherein the sulfite or bisulfite is added to the wash during the basic washing.

3. A method according to claim 1 or 2, wherein the hydrocarbon being washed is produced by the pyrolytic cracking of other hydrocarbon or hydrocarbons.

4. A method according to claim 3, wherein said other hydrocarbon or hydrocarbons is selected from ethane, propane, butane, naphtha and mixtures thereof,

5. A method according to any of claims 1 to 4, wherein the hydrocarbon being washed contains an olefin contaminated with oxygenated compound impurities.

6. A method according to any of claims 1 to 5, in which the hydrocarbons are contaminated with at least one carbonyl compound.

7. A method according to claim 6, wherein the oxygenated compounds are comprised primarily of carbonyl compounds which would polymerize to produce the fouling materials under the basic conditions of the wash.

8. A method according to claim 6 or 7, wherein the carbonyl compound or compounds is selected from an aldehyde, ketone and mixtures thereof.

9. A method according to any of claims 1 to 8, wherein the hydrocarbon being washed in a gaseous state.

10. A method according to any of claims 1 to 9, wherein the sulfite compound is an alkali metal or ammonium sulfite or bisulfite.

11. A method according to claim 10, wherein the sulfite is sodium sulfite or sodium bisulfite.

12. A method according to any of claims 1 to 11, wherein the sulfite or bisulfite thereof is employed in an amount representing a molar ratio of the sulfite to the oxygenated compound of from about 1:1 to 10:1.

13. A method according to claim 12, wherein the molar ratio is 1:1 to 3:1.